# EUROPEAN PATENT SPECIFICATION

(11) **EP 2 152 254 B1**
(45) Date of publication and mention of the grant of the patent: **22.11.2017**
(21) Application number: 08733511.3
(22) Date of filing: 16.04.2008
(51) Int. Cl.: A61K 31/05, A61P 19/08, A61P 19/10

(54) **RESVERATROL CONTAINING COMPOSITION FOR MODULATING BONE FORMATION**
RESVERATROL ENTHALTENDE ZUSAMMENSETZUNG ZUR MODULIERUNG DER KNOCHENBILDUNG
COMPOSITION COMPRENANT DU RESVERATROL POUR MODULER LA FORMATION OSSEUSE

(30) Priority: 16.04.2007 BR PI0704227
(43) Date of publication of application: 17.02.2010
(73) Proprietor: União Brasileira De Educação E Assistência - Mantenedora Da Pucrs, CEP: 90670-020 Porto Alegre - RS (BR)
(72) Inventor: SOUTO, André, Arigony, Cep: 90670-020 Porto Alegre - RS (BR); GEHRKE, Sérgio, Alexandre, Cep: 97015-00 Santa Maria - RS (BR)
(74) Representative: Capasso, Olga
(86) International application number: PCT/BR2008/000108
(87) International publication number: WO 2008/124907

(56) References cited:
- EP-A1- 1 161 944
- WO-A2-2006/081329
- US-A1- 2004 127 475
- US-B1- 6 716 883
- LI HUA SONG ET AL: "Resveratrol prevents CsA inhibition of proliferation and osteoblastic differentiation of mouse bone marrow-derived mesenchymal stem cells through an ER/NO/cGMP pathway" TOXICOLOGY IN VITRO, vol. 20, 2006, pages 915-922, XP002571298
- BÄCKESJÖ C-M ET AL: "Activation of Sirt1 decreases adipocyte formation during osteoblast differentiation of mesenchymal stem cells" JOURNAL OF BONE AND MINERAL RESEARCH, vol. 21, no. 7, 15 May 2006 (2006-05-15), pages 993-1002, XP002571300
- DATABASE MEDLINE [Online] BAGCHI D. ET AL.: 'Benefits of resveratrol in women's health', XP008115268 Database accession no. (NLM11951581) & DRUGS UNDER EXPERIMENTAL AND CLINICAL RESEARCH, SWITZERLAND vol. 27, no. 5-6, 2001, pages 233 - 248

## Description

### Field of the invention

The present invention relates to a pharmaceutical product and a process for bone regeneration and neoformation. More specifically, the pharmaceutical product of the invention comprises sirtuin modulators, such as some resveratrol derivatives. The product and process of the present invention have the advantage of accelerating the process of integration of biomaterials to the body, shortening intensively the recovery period of the injured or lost organ. The present invention has potential application in medicine, particularly to orthopaedics.

### Background of the Invention

The worldwide use of aloplastic implants in orthopaedics is becoming a constant. Aloplastic implant foreseeable condition opens new horizons to the recovery of disabled patients. In the last years, the surgical techniques as well as the materials used to achieve this goal have developed and improved. Nowadays, aloplastic implant is widely accepted in health care, particularly in dentistry. It has also been used as a solution for many unsatisfactory or unsolvable cases for professionals and patients. However, to achieve a successful implant a direct connection between living bone and titanium must exist. Thus a fundamental aspect is osteointegration, that is, a structural and functional connection between implant and bone tissue. This concept defines, in a clinical way, a biological phenomenon characterized by relative absence of soft tissue in the interface implant/bone.

One important phase of osteointegration is the osteogenic phase, characterized by bone neoformation that begins when the differentiation of the first osteoblasts in peri-implant region occurs. The preparation of the bone site to place the implant releases morphogenetic proteins forming the new bone tissue. This process constitutes the osteoinduction. The association of the functions of these proteins and the osteoblasts releases the process of bone neoformation.

In 2006, it was shown that natural product trans-resveratrol promoted the selective differentiation of mesenchymal stem cells into osteoblasts by activating sirtuin protein Sirt1 (Carl-Magnus Backesjo, et al. J. Bone and Miner Res. 2006). Trans-resveratrol modulates this protein. Sirtuin modulators can activate or deactivate changes in sirtuin functional property or biological activity. When modulator activates the protein there is a stimulation of the functional property, and when modulator deactivates the protein there is an inhibition of this property, or of the protein biological activity.

Sirtuin is a member of the sirtuin protein family deacetylases, or rather of the families Sir2, which includes yeast Sir2, *C. elegans* Sir-2.1, and human SIRT1 and SIRT2. Sirtuin protein modulators, such as resveratrol, acridine, quinoline, heterocyclic, tetrahydroquinoxaline, N-benzimidazolylalkyl amide and N,N'-dicyclic isothiourea derivatives, minimize or treat aging diseases, chronic degenerative diseases of circulatory and neurological systems, such as ocular diseases and/or disorders, psychopathologies, diabetes, cancer, obesity.

Patent literature considers some documents related to processes of osteointegration. Although none of the documents foresees or even indirectly suggests the inventive concept of the present invention, some of them are mentioned below as reference.

International patent application WO 2005/069998, "Novel sirtuin activating compounds and methods for making the same", describes the preparation of stilbene compounds for use in food, cosmetic and pharmaceutical industry. The preparation includes coupling of N-heterocyclic carbene-type ligands in the presence of a base with benzyol halide and styrene coupling partners.

International patent application WO 2006/094237, "Acridine and quinoline derivatives as sirtuin modulators", describes novel active quinoline derivatives , as sirtuin-modulating compounds useful, for example, for treating cell aging and avoiding cell death.

International patent application WO 2006/094210, "Tetrahydroquinoxalinone sirtuin modulators", describes novel tetrahydroquinoxalinone sirtuin-modulating compounds used for treating and/or preventing diseases, including cancer.

International patent application WO 2006/094209, "N-benzimidazolylalkyl-substituted amide sirtuin modulators", describes novel benzimidazol sirtuin-modulating compounds used for treating or preventing some kind of diseases, such as cancer, obesity, and neurodegenerative diseases. These compounds are used to increase cell lifespan.

International patent application WO 2006/094233, "N,n'-dicyclic isothiourea sirtuin modulators", describes novel sirtuin-modulating compounds and methods of use thereof. Sirtuin-modulating compounds may be used for increasing cell lifespan, and for treating or preventing a wide variety of diseases, such as obesity, diabetes, cardiovascular and neurodegenerative diseases.

International patent application WO 2006/127987, "Treatment of eye disorders with sirtuin modulators", describes sirtuin modulators, particularly sirtuin activators, useful to treat vision impairment. In general, the sirtuin modulators inhibit the progression of vision impairment. The invention also includes pharmaceutically acceptable formulations of sirtuin modulators, particularly ophthalmically acceptable formulations.

International patent application WO 2006/138418, "Improvement of cognitive performance with sirtuin activators", describes a method that may include administering an agent that increases protein level or sirtuin activity, such as SIRTI.

International patent application WO 2006/104586, "Methods of diagnosis and treatment of metabolic disorders", describes diagnostic methods for metabolic disorders (e.g., diabetes and obesity) and includes analyzing the level of sirtuin2 or sirtuin3 expression or activity in a sample isolated from the subject. International patent application WO 2006/102557, "Treatment of protein degradation disorders", describes a method of treating a subject suffering from or susceptible to a protein degradation disorder, (e.g., cancer). It also includes the use of a protein degradation inhibitor.

International patent application WO 2006/091582, as well as applications WO 2006/043166, WO 2005/099785, WO 2005/000373, WO 2004/103423, relate to the modification or the coating of an implant made of titanium.

International patent application WO 2005/104988, "Osteogenic implants with improved osteointegration properties", describes the impregnation of the implant surface with solution containing bone recombinant sialoprotein or recombinant His-Myc-Ek-BSP to induce differentiation of mesenchymal cells. International patent application WO 2004/024199 uses osteoinductive proteins called "Bone Morphogenetic Protein" with the same aim.

Processes using recombinant Bone Morphogenetic Proteins with this purpose are a worldwide trend. However, the industrialization of this system is costly, being an impracticable alternative nowadays.

International patent application WO 2005/099785, "Osteogenic composite matrix, method for the production thereof and implant and scaffold for tissue engineering provided with a coating formed by said osteogenic composite matrix", describes the addition of collagen components to an extracellular matrix to accelerate osteoinduction.

International patent application WO 2003/082300, "Use of carbon-2-modified-vitamin d analogs to induce the formation of new bone", describes the addition of 1α,25-dihydroxivitamin D3 derivatives to stimulate osteoblasts to the formation of a new bone.

International patent application WO 1995/013099, "Osseointegration promoting implant composition, implant assembly and method therefor", describes the use of growth factor β1 (TGF- β1) to promote osseointegration in bone implant.

Considering the reference documents mentioned above, it can be noticed that documents relating trans-resveratrol to the processes of osteointegration, or osteoinduction, or sirtuin modulators and osseointegration (osteoinduction) were not found in patent literature. Documents mentioned above refer to other processes that accelerate the process of osseointegration, without anticipating or suggesting the scope of the present invention.

The present invention shows quantitative as well as qualitative ability to stimulate the proliferation of cells with bone formation potential. The detailed description of the invention will help to understand it. Among the several technical advantages resulting from the present invention, one can mention the easy separation and purification, in a single process, of emodin and/or trans-resveratrol with high levels of purity, and the preparation of a pharmaceutical product from an alternative and more available material.

### Summary of the invention

In one aspect of the invention, being, therefore, one of its objects, a pharmaceutical product which is modulator of bone formation in vertebrates, including humans, is provided.

In other aspect of the invention, being, therefore other of its objects, a process of bone regeneration and neoformation using modulators is provided.

It is an object of the present invention to provide a product and a process that dramatically decreases the recovery period of injured or lost organ by means of body biomaterial integration .

In a preferential aspect of the invention, being, therefore, other of its objects, a pharmaceutical product comprising sirtuin modulators is provided.

In other aspect of the invention, being, therefore other of its objects, a pharmaceutical product comprising resveratrol derivatives is provided.

In other aspect of the invention, being, therefore other of its objects, a pharmaceutical product comprising trans-resveratrol is provided.

These and other objects of the present invention will be better understood and appreciated from the detailed description of the invention.

### Brief description of the Drawings

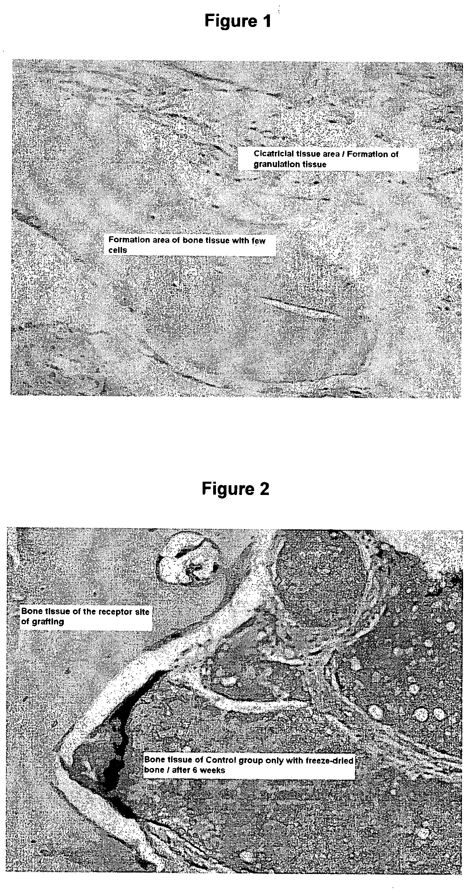

**Figure 1** shows the beginning of bone formation in a control group, whose bone formation is much slower than that of groups subject to the invention. Several areas of not hard, but in modification, tissue after six weeks are shown.
**Figure 2** shows complete filling of the surgical site. It is noticed that the process of bone tissue transformation presented, in six weeks, several areas (islands) where the development stage had not already begun. This delay shows it is necessary more time to the complete recovery of the bone structure. Test was performed in experimental group 1.

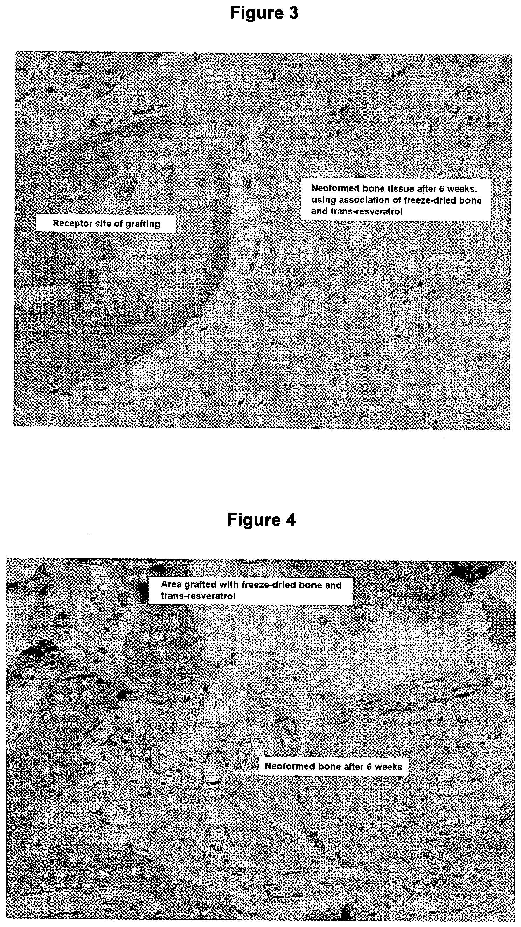

**Figures 3 and 4** show an acceleration in the process of absorbing the filling material of the surgical site, shortening the period of time to the bone neoformation. It is also observed a great increase in the proliferation of cell with osteogenic potential (osteoblasts). Regarding to the other groups, it is noticed, in six weeks, a great shortening of time in the healing process of the area where surgical site had been made. Test was performed in experimental group 2.

### Detailed description of the invention

The present invention provides a pharmaceutical product and a process for modulating bone formation. To the aims of the present description, the expression "to modulate bone formation" should be understood comprising bone regeneration and/or neoformation. The product of the invention includes bone formation modulators, particularly sirtuin protein modulators. To develop the product and the process of the invention, several experimental approaches, measurement, and technical evaluation were performed. The novel and inventive results obtained are described in further detail below.

In a first approach, inventors performed laboratory tests to induce bone tissue growth and neoformation in sites prepared in rabbit tibias. Three animals were used in pilot study. Initially, animals were submitted to standard practice of anesthesia, analgesia, and surgical preparation, that is, a trichotomy surrounding the incision site was made, thus setting up a compatible field to surgical procedure. After this procedure, antisepsis was made with iodine alcohol (iodine ethanol) and surgical fields were set up. Incision was made in two planes, one internal and the other external, with a # 15 blade scalpel. The incision made in the skin, in the medial side of the proximal tibia, was about 3.0 cm. The same procedure was carried out into the subcutaneous tissue and fascia, to the periosteum, which was cleared to expose the bone tissue. A flap was raised exposing the bone tissue. Then bores were cut with a # 6 trephine for counter-angle. Such bores were cut under abundant irrigation with 0.9% NaCl. Rotational speed was 1500 rpm and torque was 30N/cm diminishing 16/1 to prepare bone cavity, according to standard surgical protocol for bone grafting. During the procedures a Driller equipment was used, which is proper to this kind of surgery. To perform the tests, three surgical sites were prepared, with about 10 mm between them. Then only the skin was sutured. A # 6 trephine, from INP, was used to prepare surgical site. Skin was sutured with a sterile nylon suture thread, 5-0, with 75cm length and a triangular needle, from Shalon. Cavity was prepared with the trephine to do a bone hollow 5 mm diameter. Thereafter, other ones procedures were performed, as described in detail bellow. Such procedures illustrate, but not limit, preferred embodiments of the invention.

### Stage 1: Preparing, numbering, and dividing sites

In this stage, sites are prepared, divided, and numbered. After being prepared, sites were divided and numbered as site 1, site 2, site 3 and site 4. Site 1 was set up in proximal tibia. Site 2 was set up in medial tibia, and site 3 was set up in distal tibia. Experimental groups were also divided and numbered as follows:
**Group 1** - Control group (CG). In this group, surgical site was just prepared, without any filling material.
**Group 2 -** Experimental group 1 (G1) in which only freeze-dried bovine bone, medium grain (biomaterial), from Bionnovations Medical, was used for filling surgical site.
**Group 3 -** Experimental group 2 (G2) in which freeze-dried bovine bone, medium grain (biomaterial), associated to 10% of its weight of trans-resveratrol, was used for filling surgical site.

### Stage 2: Histological techniques and material processing

Six weeks after surgery, animals were killed with an overdose of Ketamina (7ml/animal). After soft tissue dissection, bone blocks and implants were removed with a saw and then sample were fixed, by immersion in neutral solution of formalin 4.0%, during thirty days.

### Stage 3: Dehydrating samples with ethanol

After thirty days, samples were washed with running water during 12 hours, and dehydrated by a series of absolute ethanol and distilled water, rigorously according to the following table.

**Table 1 - Series of alcohols for dehydration**

| **Alcohol** | **Concentration (%)** | **Time (h)** |
|---|---|---|
| Ethanol | 50 | 24 |
| Ethanol | 70 | 48 |
| Ethanol | 80 | 24 |
| Ethanol | 90 | 72 |
| Ethanol | 96 | 72 |
| Ethanol | 100 | 72 |
| Ethanol | 100 | 48 |

### Stage 4: Preparing samples and results obtained

Samples were cut after dehydration and put on slides for histological study and evaluation of results. Cuts in bone areas, which received cavities called surgical sites, were made. After laboratory assays, the following results were obtained from this pilot study:
Control group showed a beginning of bone formation, although much slower than in other experimental groups. After six weeks, several areas of not hard tissue, but tissue in modification, were found. Experimental group 1, on the other hand, presented a complete filling of the surgical site. However, its process of transformation into bone tissue presented, during the six weeks, several areas, or islands, where development stage had not begun, what hence shows that more time is necessary for the complete recovery of this bone structure. Experimental group 2 showed an excellent acceleration in the process of absorbing the filling material of surgical site, shortening the period to bone neoformation. A great increase in the proliferation of cells with osteogenic potential (osteoblasts) was also observed. Regarding the other groups, it was noticed that in six weeks a great shortening of time in the surgical site healing process occurred, as shown in figures 3 and 4. Thus, when experimental groups are compared, one can notice that there was a great increase in the healing process of experimental group 2 (G2) comparing to the other two groups, control (CG) and experimental 1 (G1). A great increase in the quantitative and qualitative proliferation of cell with bone formation potential in group G2 was also observed, resulting in the acceleration of bone neoformation.

Among the advantages provided by the invention, as mentioned above, there is acceleration in the process of integration of these biomaterials to living body (human being), with promising results. Shortening the recovery period of the injured or lost organ allows patients coming back sooner to their regular activities. There are fewer traumas to the structure related to the rehabilitated site, such as, muscles, vessels, nerves, etc. Returning this function has great psychological effect, which results in the acceptance of some types of treatment by patients. The present invention has several applications in medicine, particularly to orthopaedics, because it relates directly to bone tissue regeneration and neoformation. It is known that this field has greatly developed in the past decades both in technology and in science, presenting uncounted benefits and novel alternatives. One of the main researched aspects in this field is precisely a substance that acts as a factor of osteogenesis acceleration and stimulation, being one of the aspirations of industry related to this activity.

Those skilled in the art will immediately appreciate the important benefits brought by the present invention, such as accelerated bone regeneration and neoformation, in which the results are much more effective.

## Claims

1. Pharmaceutical composition **characterized by** comprising:
a) from 0,1% to 15% w/w of at least one sirtuin modulator chosen from the group consisting of resveratrol, trans-resveratrol and combinations thereof;
b) a material compatible with a target bone tissue chosen from the group consisting of: freeze-dried bovine bone, titanium surfaces, orthopedic prostheses and mixtures thereof; and
c) a pharmaceutically acceptable vehicle
for use in a method to promote bone regeneration and/or neoformation.

2. Pharmaceutical composition, for use according to claim 1, **characterized by** the fact that the sirtuin modulator is present at a concentration of 5% w/w.

3. Pharmaceutical composition, for use according to claim 1, **characterized by** the fact that the sirtuin modulator is present at a concentration of 10% w/w.

4. Pharmaceutical composition, for use according to any one of the above claims, **characterized by** the fact that the sirtuin modulator is trans-resveratrol and the material compatible with a target bone tissue is freeze-dried bovine bone.

5. A pharmaceutical composition **characterized by** consisting of:
a) 10% w/w of trans-resveratrol and;
b) 90% of freeze-dried bovine bone of medium grain
for use in a method to promote bone regeneration and/or neoformation.

6. A bone filing material comprising the pharmaceutical composition as defined in any one of previous claims for use in a method to promote bone regeneration and/or neoformation.

## Patentansprüche

1. Pharmazeutische Zusammensetzung, **dadurch gekennzeichnet, dass** sie Folgendes umfasst:
a) von 0,1 bis 15 Gew.-% von mindestens einem Sirtuinmodulator, ausgewählt aus der Gruppe bestehend aus Resveratrol, Trans-Resveratrol und Kombinationen davon;
b) ein Material, das mit einem Zielknochengewebe kompatibel ist, ausgewählt aus der Gruppe bestehend aus: gefriergetrocknetem, bovinem Knochen, Titanoberflächen, orthopädischen Prothesen und Mischungen davon, und
c) eine pharmazeutisch annehmbare Trägersubstanz
für die Anwendung bei einem Verfahren zur Förderung der Knochenregeneration und bzw. oder -neubildung.

2. Pharmazeutische Zusammensetzung zur Anwendung nach Anspruch 1, **gekennzeichnet durch** die Tatsache, dass der Sirtuinmodulator in einer Konzentration von 5 Gew.-% vorhanden ist.

3. Pharmazeutische Zusammensetzung zur Anwendung nach Anspruch 1, **gekennzeichnet durch** die Tatsache, dass der Sirtuinmodulator in einer Konzentration von 10 Gew.-% vorhanden ist.

4. Pharmazeutische Zusammensetzung zur Anwendung nach einem der obigen Ansprüche, **gekennzeichnet durch** die Tatsache, dass der Sirtuinmodulator Trans-Resveratrol ist und das mit einem Zielknochengewebe kompatible Material gefriergetrockneter, boviner Knochen ist.

5. Pharmazeutische Zusammensetzung, **dadurch gekennzeichnet, dass** sie Folgendes umfasst:
a) 10 Gew.-% von Trans-Resversatrol und
b) 90 % gefriergetrockneten, bovinen Knochen, mittelkörnig,
zur Anwendung bei einem Verfahren zur Förderung der Knochenregeneration und bzw. oder -neubildung.

6. Ein Knochenfüllstoffmaterial, umfassend die pharmazeutische Zusammensetzung, wie in einem der vorherigen Ansprüche definiert, zur Anwendung bei einem Verfahren zur Förderung der Knochenregeneration und bzw. oder -neubildung.

## Revendications

1. Composition pharmaceutique **caractérisée en ce qu'**elle comprend :
a) de 0,1 % à 15 % p/p d'au moins un modulateur de la sirtuine choisi dans le groupe constitué du resvératrol, du trans-resvératrol et de combinaisons de ceux-ci ;
b) un matériau compatible avec un tissu osseux cible sélectionné dans le groupe constitué de : os bovin lyophilisé, surfaces en titane, prothèses orthopédiques et mélanges de ceux-ci ; et
c) un véhicule pharmaceutiquement acceptable
pour son utilisation dans un procédé de promotion de la régénération et/ou de la néoformation osseuses.

2. Composition pharmaceutique, pour son utilisation selon la revendication 1, **caractérisée par le fait que** le modulateur de la sirtuine est présent en une concentration de 5 % p/p.

3. Composition pharmaceutique, pour son utilisation selon la revendication 1, **caractérisée par le fait que** le modulateur de la sirtuine est présent en une concentration de 10 % p/p.

4. Composition pharmaceutique, pour son utilisation selon l'une quelconque des revendications précédentes, **caractérisée par le fait que** le modulateur de la sirtuine est le trans-resvératrol et le matériau compatible avec le tissu osseux est l'os bovin lyophilisé.

5. Composition pharmaceutique **caractérisée en ce qu'**elle consiste en :
a) 10 % p/p de trans-resvératrol et ;
b) 90 % d'os bovin lyophilisé de grain moyen
pour son utilisation dans un procédé de promotion de la régénération et/ou de la néoformation osseuses.

6. Matériau de remplissage osseux comprenant la composition pharmaceutique telle que définie dans l'une quelconque des revendications précédentes pour son utilisation dans un procédé de promotion de la régénération et/ou de la néoformation osseuses.
